# EUROPEAN PATENT APPLICATION

(11) **EP 0 748 629 A1**
(43) Date of publication of application: **18.12.1996**
(21) Application number: 95900906.9
(22) Date of filing: 17.11.1994
(51) Int. Cl.: A61K 31/485, A61K 9/70

(54) **PERCUTANEOUSLY ABSORBABLE PLASTER COMPRISING ACID-ADDITION SALT OF MORPHINE**

(30) Priority: 11.03.1994 JP 40903/94
(71) Applicant: SEKISUI CHEMICAL CO., LTD., Osaka-shi, Osaka 530 (JP)
(72) Inventor: HASHIMOTO, Michiari, Mishima-gun Osaka 618 (JP); AZUMA, Masato, Mishima-gun Osaka 618 (JP); OTA, Tetsuya, Mishima-gun Osaka 618 (JP); KITAMURA, Mikiya, Osaka 571 (JP)
(74) Representative: Paul, Dieter-Alfred, Dipl.-Ing.
(86) International application number: JP9401935
(87) International publication number: WO9524197

(57) **Abstract**

A percutaneously absorbable plaster composed of a support and, formed on one side thereof, a pressure-sensitive adhesive layer comprising a pressure-sensitive adhesive, a drug and a percutaneous absorption accelerator, wherein the drug is an acid-addition salt of morphine and the accelerator comprises a compound (A) having a log P value of -0.5 to 2.0 (P being the partition coefficient of an octanol/water system). The plaster enables a pharmacologically acceptable acid-addition salt of morphine to be released uniformly and stably for long, is excellent in percutaneous penetration, and can effectively be applied to patients with pain, cough, diarrhea, and so forth.

## Description

### Technical Field

The present invention relates to a percutaneous absorption applying sheet, particularly relates to a percutaneous absorption applying sheet which can constantly release a pharmacologically acceptable acid salt of morphine for a long period of time.

### Background Art

An acid salt of morphine such as morphine hydrochloride is a drug having analgesic, antitussive or emetocathartic action, and conventionally has been administered to patients in the form of an oral preparation, injection, suppository or the like. However, there are problems that efficacy of the drug is not maintained for a long time by oral administration and the injection gives pain to patients in its administration form. Further, though the suppository improves the above-mentioned problems of the oral preparation and the injection, the suppository has problems that uncomfortableness due to the administration route is strong as well as the above-mentioned improvement effect is insufficient.

Recently, for the purpose of solving these drawbacks, a variety of studies on percutaneous absorption of morphine hydrochloride have been carried out. For example, there has been reported a percutaneous absorption preparation obtained by dissolving morphine hydrochloride in a three-component solvent of *l*-menthol/ethanol/water (Abstract of the lecture in the sixth annual meeting of the Academy of Pharmaceutical Science and Technology Japan, Abstract of the lecture in the seventh annual meeting of the same society, Extract of the lecture in the sixth transdermal therapeutic system symposium). However, there are such problems in the above-mentioned percutaneous absorption preparation that because of high volatility of ethanol, it is difficult to maintain uniform content of the drug in the percutaneous absorption preparation during preservation, further since the percutaneous absorption preparation quickly dries after application on skin, it is impossible to percutaneously absorb the drug uniformly for a long period of time.

In Japanese patent kouhyou 5007411/1990 (WO 88/01497), there is disclosed a percutaneous absorption applying sheet having a pressure-sensitive adhesive layer comprising a silicone pressure-sensitive adhesive, a morphine narcotic analgesic and a percutaneous absorption enhancer, laid on a single surface of a backing material, in which the percutaneous absorption enhancer can be a saturated or unsaturated aliphatic acid and an ester, alcohol, monoglyceride, acetate, diethanolamide and N,N-dimethylamide thereof.

Further, in Japanese Laid-open Patent Publication No. 83116/1986, there is disclosed a percutaneous absorption preparation comprising morphine and the other opioid, a percutaneous absorption enhancer and a carrier, in which the percutaneous absorption enhancer can be a saturated aliphatic alcohol or an aliphatic acid having 8 to 15 carbon atoms, or a unsaturated aliphatic alcohol or an aliphatic acid having 8 to 15 carbon atoms.

However, each of these percutaneous preparations has problems in percutaneous absorption, and thereby it is impossible to percutaneously absorb the drug uniformly for a long period, too.

The present invention has been accomplished in view of the above-mentioned problems.

An object of the present invention is to provide a percutaneous absorption applying sheet which can constantly release an acid salt of morphine uniformly for a long period of time and is excellent in percutaneous permeability of morphine.

### Disclosure of the Invention

The percutaneous absorption applying sheet of the present invention is a percutaneous absorption applying sheet having a pressure-sensitive adhesive layer comprising a pressure-sensitive adhesive, an active ingredient and a percutaneous absorption enhancer, laid on a single surface of a backing material, said active ingredient being an acid salt of morphine, and said percutaneous absorption enhancer comprising (A) an compound having a log P value (P stands for partition coefficient in octanol/water system) of from -0.5 to 2.0.

The percutaneous absorption enhancer preferably comprises (A) the above-mentioned compound and (B) an oxycarboxylic acid having 2 to 8 carbon atoms and/or a dicarboxylic acid having 2 to 8 carbon atoms.

Further, the percutaneous absorption enhancer preferably comprises (A) the above-mentioned compound, (B) the above-mentioned oxycarboxylic acid and/or dicarboxylic acid, and (C) a compound selected from the group consisting of an aliphatic acid amide which is a reaction product of an aliphatic monocarboxylic acid having 10 to 14 carbon atoms and mono or diethanol amine, an acyl sarcosine and an alkyl hydroxybenzoate having 1 to 5 carbon atoms in the alkyl group.

The backing material preferably has flexibility and has impermeability of the active ingredient. Examples of the backing material which is used in the present invention include a monolayer sheet composed of a material such as cellulose acetate, ethyl cellulose, polyethylene terephthalate, polybutylene terephthalate, ethylene-vinyl acetate-carbon monoxide copolymer, ethylene-butyl acrylate-carbon monoxide copolymer, plasticized vinyl acetate-vinyl chloride copolymer, nylon, ethylene-vinyl acetate copolymer, ethylene-methyl acrylate copolymer, plasticized polyvinyl chloride, polyethylene, polyurethane, polyvinylidene chloride, aluminum and the like, a laminate of two or more of these monolayer sheets or a laminate of the monolayer sheet and a woven fabric or unwoven fabric.

The pressure-sensitive adhesive may be a pharmaceutically acceptable material, and preferred example of the adhesive which is used in the present invention includes an adhesive having pressure sensitivity at ambient temperature such as an acrylic pressure-sensitive adhesive, a rubber pressure-sensitive adhesive or the like.

As the acrylic pressure-sensitive adhesive, there may be suitably used, for example, a polymer essentially consisting of an alkyl (meth)acrylate. This may be a copolymer of an alkyl (meth)acrylate with a copolymerizable functional monomer, a polyfunctional monomer, a vinyl compound or the like.

Preferred example of the alkyl (meth)acrylate is that having 2 to 18 carbon atoms in the alkyl group since when the number of carbon atoms in the alkyl group is smaller, pressure-sensitive adhesion decreases while cohesion rises and when the number of carbon atoms in the alkyl group is larger, cohesion decreases while pressure-sensitive adhesion rises. Examples of the alkyl (meth)acrylate include ethyl (meth)acrylate, butyl (meth)acrylate, isobutyl (meth)acrylate, hexyl (meth)acrylate, 2-ethylhexyl (meth)acrylate, octyl (meth)acrylate, isooctyl (meth)acrylate, decyl (meth)acrylate, isodecyl (meth)acrylate, lauryl (meth)acrylate, stearyl (meth)acrylate or the like.

Examples of the functional monomer are a monomer having a hydroxyl group, a monomer having a carboxyl group, a monomer having an amide group, a monomer having an amino group or the like.

As the monomer having a hydroxyl group, there may be suitably used, for example, hydroxyalkyl (meth)acrylates such as 2-hydroxyethyl (meth)acrylate, 2-hydroxypropyl (meth)acrylate and the like.

Further, as the monomer having a carboxyl group, there may be suitably used, for example, α,β-unsaturated carboxylic acids such as (meth)acrylic acid; monoalkyl maleates such as butyl maleate; maleic acid, maleic anhydride, fumaric acid, crotonic acid and the like.

As the monomer having an amide group, there may be suitably used, for example, alkyl (meth)acrylamides such as acrylamide, dimethy acrylamide, diethyl acrylamide and the like; alkyl ether methylol (meth)acrylamides such as butoxymethyl acrylamide, ethoxymethyl acrylamide and the like; diacetone acrylamide, and the like.

As the monomer having an amino group, there may be suitably used, for example, dimethylaminoethyl acrylate or the like.

The content of the functional monomer is preferably not more than 20% by weight, more preferably from 1 to 10% by weight in the pressure-sensitive adhesive, since if the content is higher, cohesion rises while pressure-sensitive adhesion lowers.

Examples of the polyfunctional monomer are 1,6-hexane glycol dimethacrylate, tetraethylene glycol diacrylate, trimethylolpropane triacrylate, divinylbenzene, divinyltoluene, diallyl phthalate, diallyl maleate, diallyl adipate, diallyl glycolate, triallyl isocyanurate, diethylene glycol bisallyl carbonate or the like.

The content of the polyfunctional monomer is preferably from 0.005 to 0.5% by weight in the pressure-sensitive adhesive, since when the content is lower, cohesion improvement effect is scarcely recognized and when the content is higher, cohesion rises while pressure-sensitive adhesion lowers.

Examples of the vinyl compound are vinyl acetate, styrene, α-methylstyrene, N-vinyl-2-pyrrolidone, vinyl chloride, acrylonitrile, ethylene, propylene, butadiene or the like.

The content of the vinyl compound is preferably not more than 50% by weight, more preferably not more than 40% by weight in the pressure-sensitive adhesive, since if the content is higher, cohesion rises while pressure-sensitive adhesion lowers.

Further, to the acrylic pressure-sensitive adhesive, may be added a tackifier, a filler and the like for regulation of the pressure-sensitive adhesion within the pharmaceutically acceptable range, if necessary.

The example of the tackifier includes rosin resin, terpene resin, cumarone-indene resin, petroleum resin, terpene-phenol resin and the like, and preferred example is glycerin ester of hydrogenated rosin "ESTERGUM H" (manufactured by Arakawa Chemical, Ltd.) or the like.

The content of the tackifier is from 0 to 200 parts by weight, preferably from 20 to 100 parts by weight based on 100 parts by weight of the acrylic pressure-sensitive adhesive. The content of the glycerin ester of a hydrogenated rosin is preferably from 1 to 200 parts by weight, more preferably from 30 to 150 parts by weight based on 100 parts by weight of the acrylic pressure-sensitive adhesive.

Examples of the filler include hydrophobic silica, hydrophilic silica, calcium carbonate, titanium oxide, polyvinyl pyrrolidone and the like.

The acrylic pressure-sensitive adhesive is prepared by, for example, blending the above-mentioned monomers in the presence of a polymerization initiator followed by carrying out solution polymerization.

The rubber pressure-sensitive adhesive is a pressure-sensitive adhesive essentially consisting of a rubber elastomer, and a tackifier, a softening agent, an aging inhibitor and the like are added, if necessary.

Examples of the rubber elastomer include cis-1,4-isoprene (natural rubber), trans-1,4-isoprene, polyisobutylene, polybutadiene, styrene-butadiene copolymer, styrene-isoprene copolymer, styrene-isoprene-styrene block copolymer, styrene-butadiene-styrene block copolymer, styrene-olefin-styrene block copolymer, styrene-isoprene-butylene block copolymer, polyvinyl ether, polyurethane, silicone rubber or the like.

Examples of the tackifier include terpene resins such as rosin, hydrogenated rosin, disproportionated rosin, rosin ester, α-pinene, β-pinene and the like, terpene-phenol resins, petroleum resin, alkyl-phenol resin, xylene resin, cumarone resin, cumarone-indene resin and the like.

The content of the tackifier is preferably from 20 to 200 parts by weight based on 100 parts by weight of the rubber pressure-sensitive adhesive.

Examples of the softening agent are oils such as process oil, palm oil, cottonseed oil, coconut oil, castor oil and the like; polybutene, liquid isobutylene, liquid polyacrylate, beeswax, carnauba wax, lanolin, and the like.

Preferred pressure-sensitive adhesive is the acrylic pressure-sensitive adhesive because it has a high saturated solubility of the active ingredient, and there are suitably used (a) a copolymer including as monomer units an alkyl (meth)acrylate and N-vinyl-2-pyrrolidone, for example, a copolymer of an alkyl (meth)acrylate, N-vinyl-2-pyrrolidone and a polyfunctional monomer (the polymerization ratio by weight is, for example, as follows: 65 to 99 parts of an alkyl (meth)acrylate; 1 to 35 parts of N-vinyl-2-pyrrolidone; 0 to 0.5 part of a polyfunctional monomer), and (b) a copolymer including as monomer units a plurality of alkyl (meth)acrylates, for example, a copolymer of a plurality of alkyl (meth)acrylates and a polyfunctional monomer, more especially a copolymer of 2-ethylhexyl methacrylate, 2-ethylhexyl acrylate, dodecyl methacrylate and a polyfunctional monomer (the polymerization ratio by weight is, for example, as follows: 100 parts of 2-ethylhexyl methacrylate; 5 to 30 parts of 2-ethylhexyl acrylate; 5 to 30 parts of dodecyl methacrylate; 0 to 0.5 part of a polyfunctional monomer).

The acid salt of morphine, which is used as the active ingredient of the present invention, for example, may be morphine hydrochloride, morphine sulfate and the like. The content of the acid salt of morphine is preferably from 0.1 to 65 parts by weight, more preferably from 3 to 60 parts by weight based on 100 parts by weight of the pressure-sensitive adhesive, since when the content is lower, drug efficacy lowers, and if required drug efficacy is to be obtained, applying area enlarges to make application for a long period difficult, and when the content is higher, pressure-sensitive adhesion of the pressure-sensitive adhesive lowers to make application for a long period difficult.

Next, explanation is made for the percutaneous absorption enhancer which is the feature of the present invention.

The log P value of the compound (A) is a value which represents hydrophobicity (in the formula, P stands for partition coefficient in octanol/water system). Since percutaneous absorption enhancing effect of the active ingredient lowers in either case when the log P value decreases or when the value increases, the log P value is selected within the range of from -0.5 to 2.0. The more preferable range of this value is from -0.3 to 1.8.

Here, a lower log P value stands for hydrophilicity and a higher log P value stands for hydrophobicity. The log P value is calculated (see EUR. J. MED. CHEM.-CHIMICA THEPAPEUTICA, JULY-AUGUST, 1974-9, No. 4, p 361-375).

Examples of the compound (A) include a polyoxyethylene lauryl ether having 6 to 12 oxyethylene segments which are repeating units, a polyoxyethylene cetyl ether having 14 to 16 oxyethylene segments, an aliphatic alcohol having 4 to 6 carbon atoms, an aliphatic monocarboxylic acid having 2 to 5 carbon atoms or the like, and they may be used alone or in combination of two or more.

Examples of the aliphatic alcohol having 4 to 6 carbon atoms include butyl alcohol, isobutyl alcohol, t-butyl alcohol, pentyl alcohol, isopentyl alcohol, hexyl alcohol or the like.

The aliphatic monocarboxylic acid having 2 to 5 carbon atoms is exemplified by acetic acid, propionic acid, butyric acid, valeric acid or the like.

The content of the compound (A) is preferably from 0.1 to 15 parts by weight, more preferably from 1 to 12 parts by weight based on 100 parts by weight of the pressure-sensitive adhesive, since when the content is lower, percutaneous absorption enhancing effect of the active ingredient lowers and when the content is higher, compatibility with the pressure-sensitive adhesive deteriorates to lower pressure-sensitive adhesion of the pressure-sensitive adhesive.

Preferred oxycarboxylic acid and dicarboxylic acid (B) are those having 2 to 8 carbon atoms, since when the number of carbon atoms is larger, percutaneous absorption enhancing effect of the active ingredient lowers.

Examples of the oxycarboxylic acid include lactic acid, glyceric acid, tartaric acid, citric acid or the like. Examples of the dicarboxylic acid include a saturated aliphatic dicarboxylic acid such as oxalic acid, malonic acid, succinic acid, glutaric acid, adipic acid, pimelic acid, suberic acid or the like; a unsaturated aliphatic dicarboxylic acid such as fumaric acid, maleic acid or the like; an aromatic dicarboxylic acid such as phthalic acid, isophthalic acid, terephthalic acid or the like; malic acid, or the like. Preferred example of the oxycarboxylic acid and dicarboxylic acid (B) is lactic acid.

The content of the oxycarboxylic acid or dicarboxylic acid (B) is preferably from 0.1 to 15 parts by weight, more preferably from 1 to 7 parts by weight based on 100 parts by weight of the above-mentioned pressure-sensitive adhesive, since when the content is lower, the percutaneous absorption enhancing effect lowers and when the content is higher, compatibility with the pressure-sensitive adhesive deteriorates to lower pressure-sensitive adhesion of the pressure-sensitive adhesive.

When the percutaneous absorption enhancer consists of the above-mentioned organic compound (A) and the above-mentioned oxycarboxylic acid and/or dicarboxylic acid (B), the contents of the above-mentioned organic compound (A) and the above-mentioned oxycarboxylic acid and/or dicarboxylic acid (B) are preferably from 0.1 to 15 parts by weight, respectively, more preferably from 1 to 12 parts by weight based on 100 parts by weight of the pressure-sensitive adhesive.

The compound (C) is a compound selected from the group consisting of an aliphatic acid amide which is a reaction product of an aliphatic monocarboxylic acid having 10 to 14 carbon atoms and mono or diethanol amine, an acylsarcosine and an alkyl hydroxybenzoate having 1 to 5 carbon atoms in the alkyl group, or a combination thereof.

Preferred examples of the aliphatic acid amide include capric acid di(mono)ethanol amide, lauric acid di(mono)ethanol amide, palmitic acid di(mono)ethanol amide or the like, especially, lauric acid di(mono)ethanol amide.

Preferred examples of the acylsarcosine include N-lauroyl sarcosine, coconut fatty acid sarcosine, N-stearoyl sarcosine, N-oleoyl sarcosine, N-palmitoyl sarcosine or the like, especially, N-lauroyl sarcosine.

Preferred examples of the alkyl hydroxybenzoate include methyl p-(o- or m-)hydroxybenzoate, ethyl p-(o- or m-)hydroxybenzoate, propyl p-(o- or m-)hydroxybenzoate, isopropyl p-(o- or m-)hydroxybenzoate, butyl p-(o- or m-)hydroxybenzoate or the like, especially, propyl p-(o- or m-)hydroxybenzoate.

The content of the compound (C) is preferably from 0.1 to 15 parts by weight, more preferably from 1 to 7 parts by weight based on 100 parts by weight of the pressure-sensitive adhesive, since when the content is lower, the percutaneous absorption enhancing effect of the active ingredient lowers and when the content is higher, pressure-sensitive adhesion of the pressure-sensitive adhesive lowers and at the same time skin stimulation occurs owing to excessive migration of the compound (C) into the skin.

When the percutaneous absorption enhancer consists of the above-mentioned organic compound (A), the above-mentioned oxycarboxylic acid and/or dicarboxylic acid (B) and the above-mentioned compound (C), the contents of the above-mentioned organic compound (A), the above-mentioned oxycarboxylic acid and/or dicarboxylic acid (B) and the above-mentioned compound (C) are preferably from 0.1 to 15 parts by weight, respectively, more preferably from 1 to 12 parts by weight based on 100 parts by weight of the pressure-sensitive adhesive.

The preferred percutaneous absorption applying sheet of the present invention is that in which the above-mentioned pressure-sensitive adhesive layer comprises 100 parts by weight of the pressure-sensitive adhesive, 0.1 to 65 parts by weight of the acid salt of morphine and 0.1 to 45 parts by weight of the percutaneous absorption enhancer.

The preferred percutaneous absorption applying sheet of the present invention is usually equipped with a release paper on the surface of the applying layer to protect the surface until use. As the release paper, a polyethylene terephthalate film treated with silicon is often used. However, this is not restrictive. The thickness of the release paper is not more than 1000 µm, preferably from 30 to 200 µm.

The thickness of the pressure-sensitive adhesive layer is not restricted, but it is preferably from 20 to 200 µm, more preferably from 30 to 100 µm, since when it is thinner, the active ingredient has to be added in a large amount to give drug efficacy, and pressure-sensitive adhesion of the pressure-sensitive adhesive lowers and when it is thicker, the active ingredient in the pressure-sensitive adhesive layer is not effectively utilized just to raise cost and performance is not improved.

The construction of the percutaneous absorption applying sheet of the present invention is as previously described, and any method may be adopted for its production. For example, there may be applied the following methods; a method which comprises mixing a pressure-sensitive adhesive such as a solvent type or emulsion type pressure-sensitive adhesive, an active ingredient and a percutaneous absorption enhancer, and applying the resulting mixture on a backing material followed by drying it; a method which comprises applying the above-mentioned mixture on a release paper and drying it, then laminating the resulting pressure-sensitive adhesive layer on a backing material; a method which comprises mixing a hot-melt type pressure-sensitive adhesive, an active ingredient and a percutaneous absorption enhancer, and applying the resulting mixture on a backing material followed by drying it; and the like.

The acid salt of morphine, which is the active ingredient of the present invention, is a relatively unstable compound, and is decomposed with lapse of time especially when oxygen coexists, therefore, in preservation of the percutaneous absorption applying sheet of the present invention, it is preferable to shut out oxygn or to preserve the percutaneous absorption applying sheet with a deoxygenating agent. As the packaging material, there may be used a sheet or a film having a low oxygen permeability, preferably of not more than 100 cm³/m² · atm · 24 hrs (25°C), more preferably of not more than 20 cm³/m² · atm · 24 hrs (25°C). Examples of such film include an aluminium sheet, an aluminium sheet of which surface is covered with polyethylene, polyethylene terephthalate and the like, a polyvinylidene chloride film, a polyvinylidene chloride-polyethylene laminated film and the like, and they are commercially available under the name of VARIARON-CX (manufactured by Asahi Chemical, Co., Ltd.), PAIRFLEX (manufactured by Kureha Chemical Co., Ltd.), KEIFLEX (manufactured by Kureha Chemical Co., Ltd.), BOBURON (manufactured by Nichigou Film, Ltd.), EMBLER-OV (manufactured by UNITIKA, Ltd.), REIFAN NO (manufactured by Toray Synthetic Film, Ltd.) and the like.

Examples of the deoxygenating agent include powder, granule, tablet and the like such as activated iron oxide, hydrosulfite, ascorbic acid, butyl hydroxy toluene and the like, and they are commercially available under the name of AGELESS Z-20 (manufactured by Mitsubishi Gas Chemical, Co., Inc.), Freshness retaining agent F (manufactured by Toppan Printing, Co., Ltd.) and the like.

### Brief Description of the Drawings

Fig. 1 is a perspective view of the diffusion cell used in Skin Permeability Test.

Fig. 2 is a graph showing change with lapse of time in the concentration of morphine in plasma.

### Best mode for carrying out the invention

Then, examples of the present invention are described.

### Synthesis of the acrylic pressure-sensitive adhesive (a)

A separable flask equipped with a stirring apparatus and a cooling apparatus was charged with 302.0 g (65 % by mole) of 2-ethylhexyl acrylate, 98.0 g (35% by mole) of N-vinyl-2-pyrrolidone and 40.0 mg of 1,6-hexane glycol dimethacrylate, further, thereto was added 400.0 g of ethyl acetate to regulate monomer concentration to 50% by weight. This solution was heated to 60°C under nitrogen atmosphere, and a polymerization reaction was conducted for 12 hours with gradual addition of 240 g of ethyl acetate and a solution of 2 g of lauroyl peroxide dissolved in 100 g of cyclohexane, to obtain an ethyl acetate solution of an acrylic pressure-sensitive adhesive having a solid content of 35% by weight [hereinafter referred to as acrylic pressure-sensitive adhesive (a)].

### Synthesis of the acrylic pressure-sensitive adhesive (b)

The same procedure as the above-mentioned synthesis procedure of the acrylic pressure-sensitive adhesive (a) was repeated except that the monomer charging amounts were changed to 36.4 g (10% by mole) of 2-ethylhexyl acrylate, 313.3 g (80% by mole) of 2-ethylhexyl methacrylate, 50.2 g (10% by mole) of dodecyl methacrylate and 52.0 mg of 1,6-hexane glycol dimethacrylate, to obtain an ethyl acetate solution of an acrylic pressure-sensitive adhesive having a solid content of 35% by weight [hereinafter referred to as acrylic pressure-sensitive adhesive (b)].

The formulations of the monomers in the pressure-sensitive adhesive syntheses are shown in Table 1.

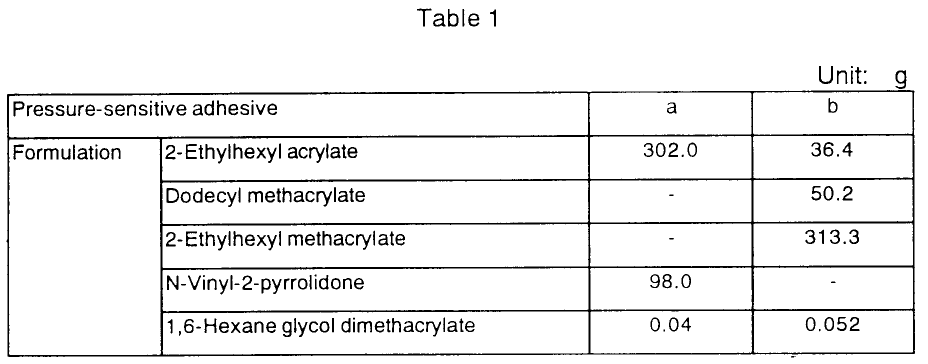

### Example 1

A vessel was charged with 100 parts by weight (in terms of solid) of the acrylic pressure-sensitive adhesive (a), 27 parts by weight of morphine hydrochloride and 6.7 parts by weight of polyoxyethylene(9) lauryl ether, then thereto was further added ethyl acetate to regulate solid concentration to 30% by weight, and the whole solution was uniformly mixed to obtain a coating liquid. This coating liquid was coated on a polyester terephthalate film (thickness is 48 µm) which had been treated with silicon, and the film was dried at 60°C in a Gear oven for 30 minutes to form a pressure-sensitive adhesive layer having a thickness of 80 µm. Then, the resulting pressure-sensitive adhesive layer was laminated on the surface of an ethylene-vinyl acetate copolymer layer of a backing material having a thickness of 38 µm (the backing material is a laminated film consisting of polyethylene terephthalate and the ethylene-vinyl acetate copolymer) to obtain a percutaneous absorption applying sheet of the present invention.

### Examples 2 to 30

The same procedure as Example 1 was repeated to obtain a percutaneous absorption applying sheet of the present invention, except that the acrylic pressure-sensitive adhesive, the active ingredient and the percutaneous absorption enhancer shown in Table 2 or 3 were charged into a vessel in given amounts, thereto was further added ethyl acetate to regulate solid concentration to 30% by weight, and the whole solution was uniformly mixed to obtain a coating liquid.

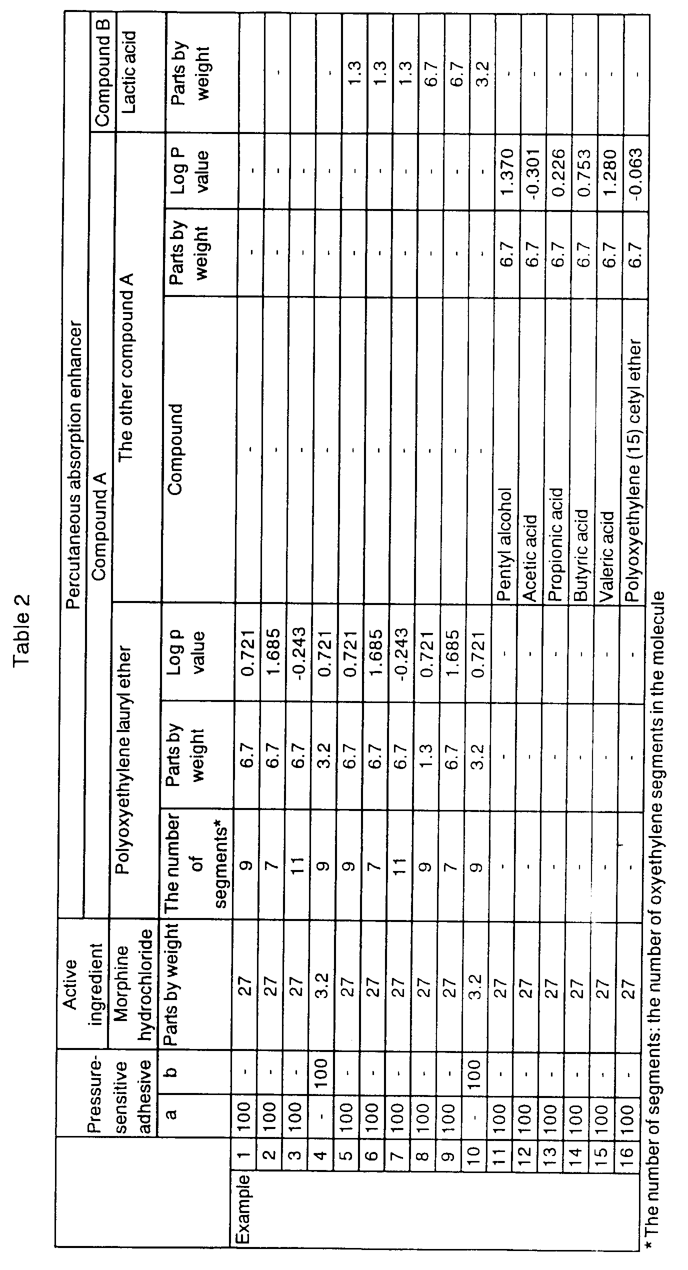

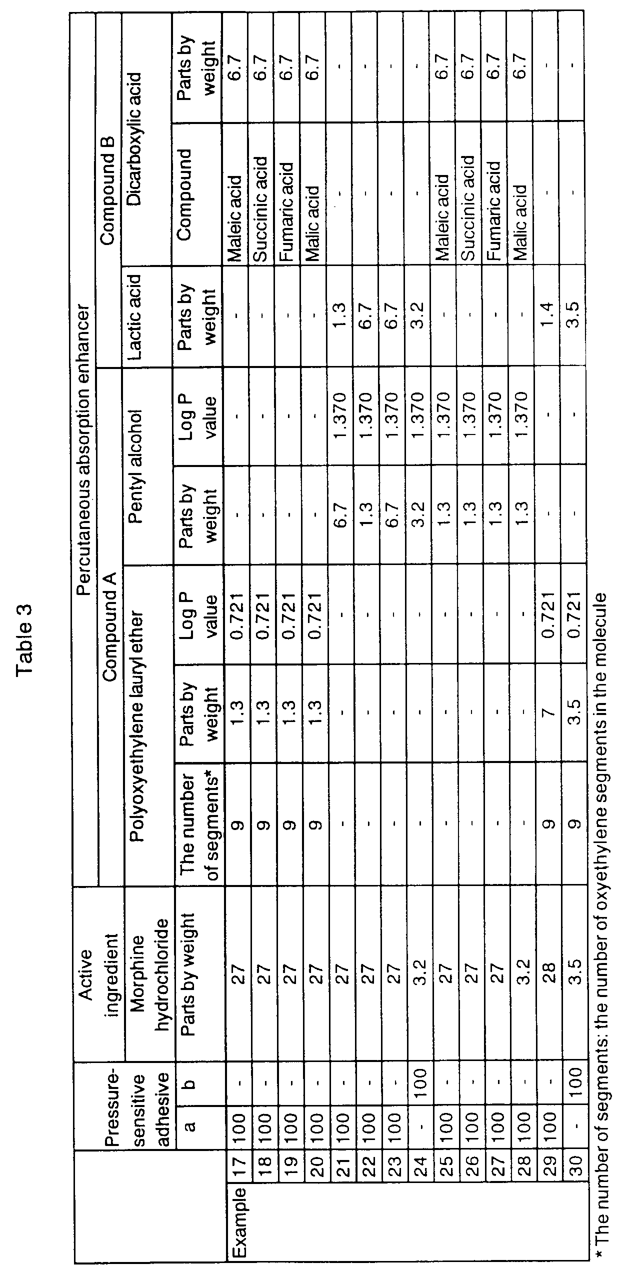

### Comparative Examples 1 to 8

The same procedure as Example 1 was repeated to obtain a percutaneous absorption applying sheet, except that the acrylic pressure-sensitive adhesive, the active ingredient and the percutaneous absorption enhancer shown in Table 4 were charged into a vessel in given amounts, thereto was further added ethyl acetate to regulate solid concentration to 30% by weight, and the whole solution was uniformly mixed to obtain a coating liquid.

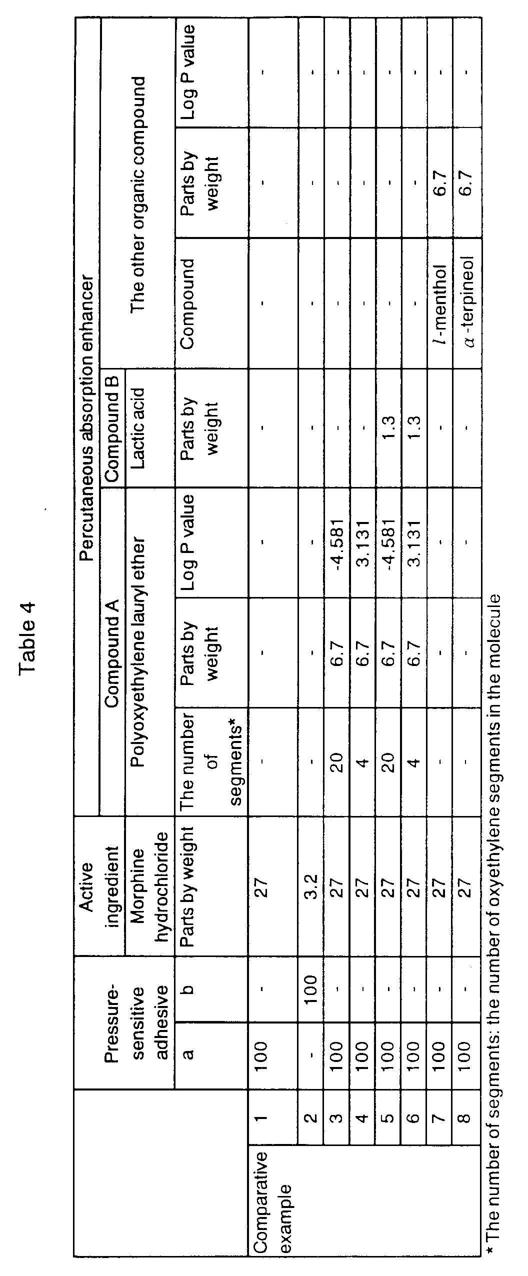

### Example 31

A vessel was charged with 100 parts by weight (in terms of solid) of the acrylic pressure-sensitive adhesive (a), 28 parts by weight of morphine hydrochloride, 7 parts by weight of polyoxyethylene(9) lauryl ether [compound(A)], 1.4 parts by weight of lactic acid [compound (B)] and 4.2 parts by weight of lauric acid diethanol amide [compound (C)], then thereto was added ethyl acetate to regulate solid concentration to 30% by weight, and the whole solution was uniformly mixed to obtain a coating liquid. This coating liquid was coated on a polyester terephthalate film (thickness is 48 µm) which had been treated with silicon, and the film was dried at 60°C in a Geer oven for 30 minutes to form a pressure-sensitive adhesive layer having a thickness of 80 µm. Then, the resulting adhesive layer was laminated on the surface of an ethylene-vinyl acetate copolymer layer of a backing material having a thickness of 38 µm (the backing material is a laminated film consisting of polyethylene terephthalate and the ethylene-vinyl acetate copolymer) to obtain a percutaneous absorption applying sheet of the present invention.

### Examples 32 to 40

The same procedure as Example 31 was repeated to obtain a percutaneous absorption applying sheet of the present invention, except that the acrylic pressure-sensitive adhesive, the active ingredient and the percutaneous absorption enhancer shown in Table 5 were charged into a vessel in given amounts, thereto was further added ethyl acetate to regulate solid concentration to 30% by weight, and the whole solution was uniformly mixed to obtain a coating liquid.

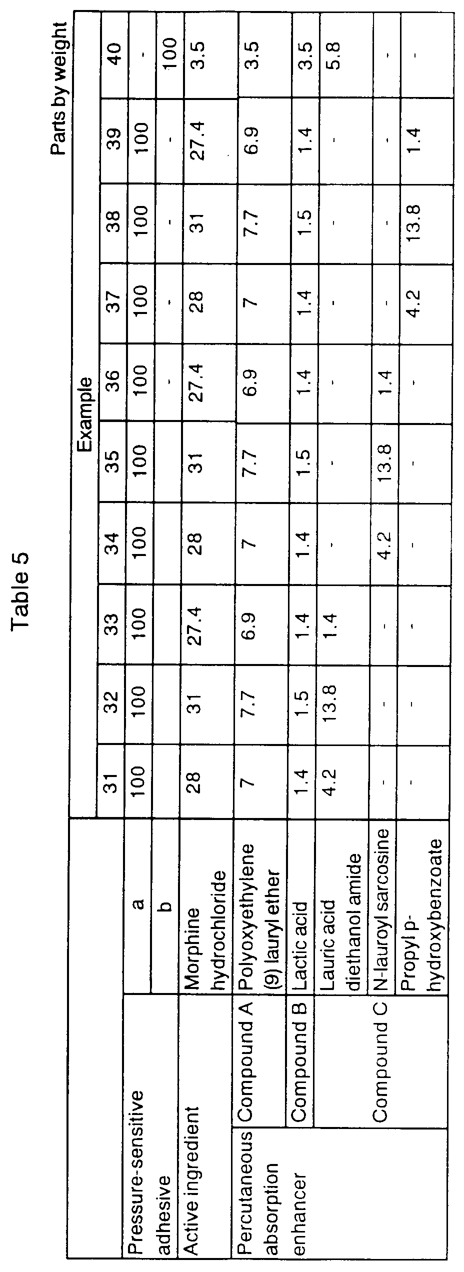

### Examples 41 to 54

The same procedure as Example 31 was repeated to obtain a percutaneous absorption applying sheet of the present invention, except that the acrylic pressure-sensitive adhesive, the tackifier, the active ingredient and the percutaneous absorption enhancer shown in Table 6 or 7 were charged into a vessel in given amounts, thereto was further added ethyl acetate to regulate solid concentration to 30% by weight, and the whole solution was uniformly mixed to obtain a coating liquid.

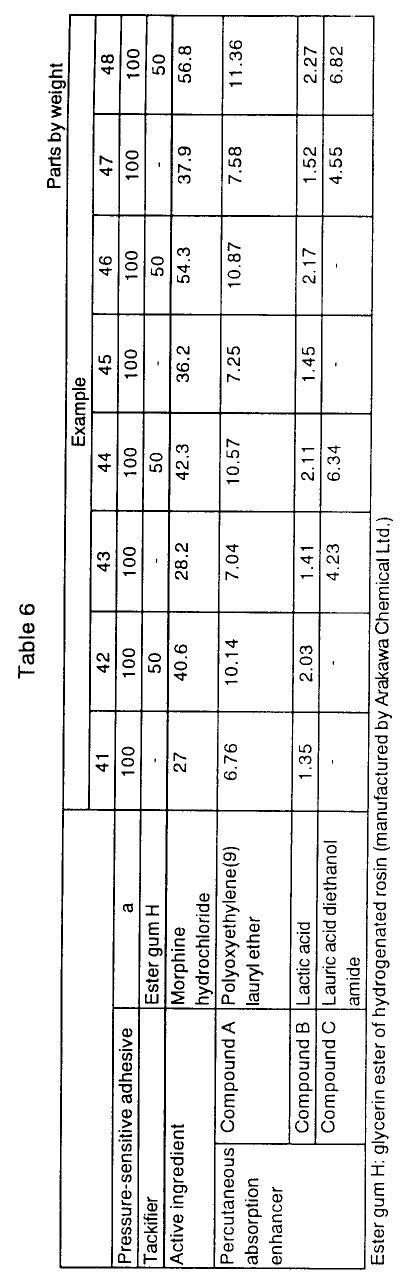

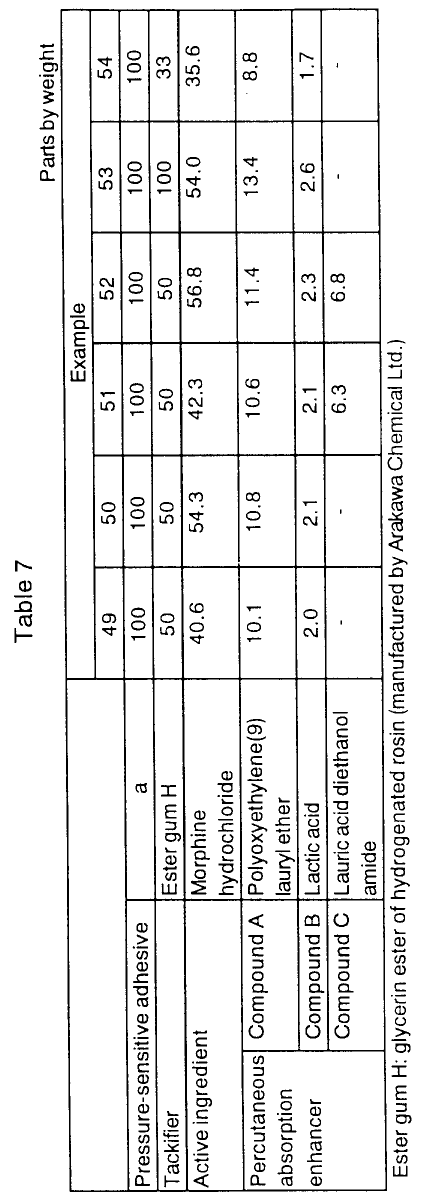

### Skin Permeability Test

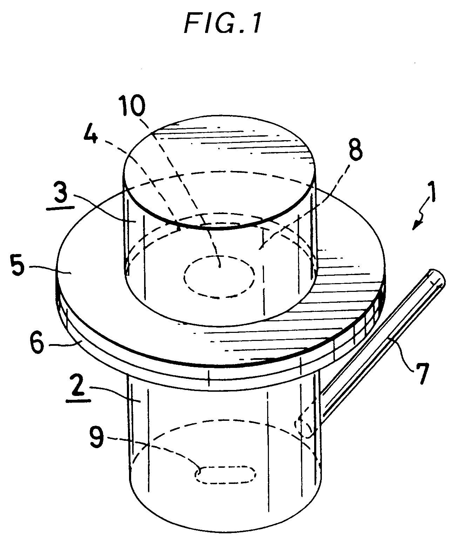

Using a Franz type diffusion cell (1) shown in Fig. 1, a skin permeability test was conducted to measure skin permeation (µg) of the active ingredient with respect to the percutaneous absorption applying sheets obtained in the above-mentioned Examples and Comparative Examples. In Fig. 1, the diffusion cell is composed of a reverse hat form receptor vessel (2 and a hat form donor vessel (3) placed on the vessel (2). On the side wall of the receptor vessel (2), a laterally projecting sampling inlet (7) is fixed and in the receptor vessel (2), a magnet stirrer (9) is placed.

The donor vessel (3) is placed on the receptor vessel (2) so that each opening faces each other, and by fitting the flange (5) of the donor vessel (3) to the flange (6) of the receptor vessel (2), the receptor vessel (2) and the donor vessel (3) are piled up in air tight fashion.

A hairless mouse (6 weeks old, male) was slaughtered by dislocation of vertebrae cervicales, and back skin was immediately peeled, panniculus and muscularis were removed to get a skin piece (8) of about 5 cm × 5 cm.

This skin piece (8) was sandwiched between the flange (5) and the flange (6) of the diffusion cell (1), to completely close the opening (4) of the donor vessel (3) with the skin piece (8).

The percutaneous absorption applying sheet obtained in Examples or Comparative Examples was cut into a circle (3.14 cm²), the pressure-sensitive adhesive layer of the resulting test piece (10) was applied on the center of the skin piece (8) in the opening (4) of the donor vessel (3).

The receptor vessel (2) was filled with receptor solution, the receptor vessel (2) was placed in a thermostat chamber kept at 37°C, and the magnetic stirrer (9) was rotated with a magnetic stirring apparatus to stir the solution. Twenty four hours after initiation of the test, 1 ml of the receptor solution was sampled through the sampling inlet (7), the active ingredient content in this sampling solution was measured by high performance chromatography to determine a skin permeation per 3.14 cm² of the test piece. In sampling of the receptor liquid, the receptor liquid was refilled after sampling. The test was repeated three times for each applying sheet, and an average of measured values was calculated. Thus obtained values are shown in Tables 8 and 9.

Here, the receptor solution was prepared by dissolving 5 × 10⁻⁴ mol of NaH₂PO₄, 2 × 10⁻⁴ mol of Na₂HPO₄, 1.5 × 10⁻¹ mol of NaCl and 10 mg of gentamycin in 500 ml of distilled water, adding a 0.1 N aqueous NaOH solution to the resulting solution to regulate pH to 7.2, and further adding distilled water to make the volume up to 1000 ml.

**Table 8**

| Skin permeation (µg) | | | Skin permeation (µg) | | |
|---|---|---|---|---|---|
| Example | 1 | 200 | Example | 20 | 2,000 |
| | 2 | 180 | | 21 | 1,600 |
| | 3 | 160 | | 22 | 1,500 |
| | 4 | 150 | | 23 | 3,000 |
| | 5 | 3,000 | | 24 | 900 |
| | 6 | 2,700 | | 25 | 1,400 |
| | 7 | 2,500 | | 26 | 1,300 |
| | 8 | 2,300 | | 27 | 1,500 |
| | 9 | 3,500 | | 28 | 1,400 |
| | 10 | 1,900 | | 29 | 3,020 |
| | 11 | 250 | | 30 | 1,900 |
| | 12 | 180 | Comparative example | 1 | 90 |
| | 13 | 180 | | 2 | 70 |
| | 14 | 170 | | 3 | 90 |
| | 15 | 170 | | 4 | 80 |
| | 16 | 210 | | 5 | 90 |
| | 17 | 2,000 | | 6 | 100 |
| | 18 | 1,800 | | 7 | 100 |
| | 19 | 2,100 | | 8 | 110 |

**Table 9**

| | | Skin permeation (µg) | Pressure-sensitive adhesion (g) |
|---|---|---|---|
| Example | 31 | 4,510 | - |
| | 32 | 5,970 | - |
| | 33 | 3,650 | - |
| | 34 | 4,640 | - |
| | 35 | 6,245 | - |
| | 36 | 3,890 | - |
| | 37 | 4,430 | - |
| | 38 | 4,690 | - |
| | 39 | 3,220 | - |
| | 40 | 3,800 | - |
| | 49 | 2,800 | 190 |
| | 50 | 3,400 | 150 |
| | 51 | 4,500 | 150 |
| | 52 | 5,890 | 120 |
| | 53 | - | 340 |
| | 54 | - | 160 |

### Preservation test

The percutaneous absorption applying sheets obtained in Examples 1 to 28 were cut into a size of 9 cm × 9 cm, the resulting test pieces were packaged in the following packaging material and were preserved at 60°C for one month, then, the test pieces were taken out of the packaging material, coloring degree of the pressure-sensitive adhesive layer of each applying sheet was visually observed.

Packaging method; a laminated film was cut into a size of 10 cm × 10 cm, the laminated film comprising an aluminium film (thickness is 9 µm) on one surface of which a polyethylene terephthalate film (thickness is 12 µm) and a polyethylene film (thickness is 12 µm) are laminated and on another surface of which a polyethylene film (thickness is 40 µm) is laminated. A pair of resulting cut pieces were put together so that each polyethylene film faces each other and periphery portion of the cut pieces was heat sealed such that one test piece and one deoxygenating agent (Ageless Z-20 manufactured by Mitsubishi Gas Chemical Co., Inc.; oxygen absorption ability is 20 cm³) were enclosed.

As the result, an excellent preservation ability was recognized, and no coloring was observed in any percutaneous absorption applying sheet of Examples 1 to 28.

### Pressure-sensitive adhesion test

Pressure-sensitive adhesion was measured with respect to each percutaneous absorption applying sheet obtained in Examples 49 to 54. This measuring was conducted according to 180° pealing method prescribed in JIS Z0237 "Test method for pressure-sensitive adhesion tape · pressure-sensitive adhesion sheet". Here, a test piece having a width of 15 mm was used. The test was repeated three times for each applying sheet, and an average of resulting measured values was calculated. Thus obtained values are shown in Table 9.

The same procedures were repeated as Examples 49 to 52 to obtain percutaneous absorption applying sheets (49') (50') (51')(52'), except that the tackifiers were not used and the contents of the pressure-sensitive adhesives were respectively increased to 150 parts by weight in Examples 49 to 52. With respect to these applying sheets, pressure-sensitive adhesion was measured as previously described. As the result, pressure-sensitive adhesion was 80 g for the applying sheet (49'), 50 g for the applying sheet (50'), 55 g for the applying sheet (51') and 30 g for the applying sheet (52').

### Percutaneous absorption test

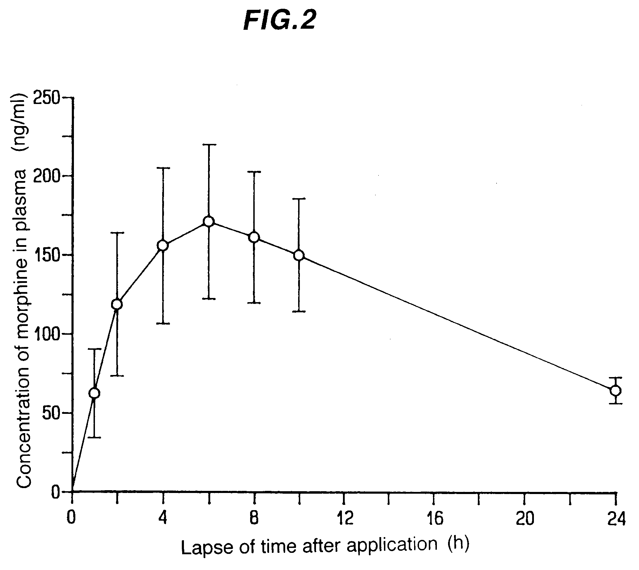

A male hairless rat (body weight is about 170 g) of which back was shaved with an electric hair clippers or electric shaver 24 hours before application, was offered for a percutaneous absorption test. Three percutaneous absorption applying sheets (6.7 cm²/one sheet) obtained in Example 49 were applied on the shaved back, and they were fixed with a gauze and an adhesive tape. Then, 1, 2, 4, 6, 8, 10 and 24 hours after application, 0.2 ml of blood was taken from jugular veins, and after separation of plasma, morphine concentration in plasma was measured by HPLC according to the following condition. The test was repeated with respect to 10 samples of the hairless rat. The results are shown in Fig. 2 (the value is average ± standard error).

### 〈HPLC condition〉

- column:: YMC Pack ODS-A AM-312
- mobile phase:: pH 2.1 phosphoric acid buffer solution/acetonitrile/methanol (74/24/2)
- flow rate:: 2.0 ml/minute
- detector:: electrochemical detector

### Industrial Applicability

The present invention provides a percutaneous absorption applying sheet which can constantly release a pharmacologically acceptable acid salt of morphine for a long period of time and is excellent in percutaneous permeability. The percutaneous absorption applying sheet of the present invention can be effectively applied to patients having a symptom such as pain, cough, diarrhea or the like.

## Claims

1. A percutaneous absorption applying sheet having a pressure-sensitive adhesive layer comprising a pressure-sensitive adhesive, an active ingredient and a percutaneous absorption enhancer, laid on a single surface of a backing material, said active ingredient being an acid salt of morphine, and said percutaneous absorption enhancer comprising (A) an organic compound having a log P value (P stands for partition coefficient in octanol/water system) of from -0.5 to 2.0.

2. The percutaneous absorption applying sheet according to claim 1, wherein said organic compound (A) is a compound selected from the group consisting of a polyoxyethylene lauryl ether having 6 to 12 oxyethylene segments, a polyoxyethylene cetyl ether having 14 to 16 oxyethylene segments, an aliphatic alcohol having 4 to 6 carbon atoms and an aliphatic monocarboxylic acid having 2 to 5 carbon atoms, or a mixture thereof.

3. The percutaneous absorption applying sheet according to claim 1 or 2, wherein the content of said organic compound (A) is 0.1 to 15 parts by weight based on 100 parts by weight of the pressure-sensitive adhesive.

4. The percutaneous absorption applying sheet according to claim 1, wherein said percutaneous absorption enhancer comprises (A) the compound having a log P value (P stands for partition coefficient in octanol/water system) of from -0.5 to 2.0, and (B) an oxycarboxylic acid having 2 to 8 carbon atoms and/or a dicarboxylic acid having 2 to 8 carbon atoms.

5. The percutaneous absorption applying sheet according to claim 4, wherein the contents of said organic compound (A) and said oxycarboxylic acid and/or dicarboxylic acid (B) are from 0.1 to 15 parts by weight based on 100 parts by weight of the pressure-sensitive adhesive, respectively.

6. The percutaneous absorption applying sheet according to claim 1, wherein said percutaneous absorption enhancer comprises (A) the compound having a log P value (P stands for partition coefficient in octanol/water system) of from -0.5 to 2.0, (B) the oxycarboxylic acid having 2 to 8 carbon atoms and/or the dicarboxylic acid having 2 to 8 carbon atoms, and (C) a compound selected from the group consisting of an aliphatic acid amide which is a reaction product of an aliphatic monocarboxylic acid having 10 to 14 carbon atoms and mono or diethanolamine, an acyl sarcosine and an alkyl hydroxybenzoate having 1 to 5 carbon atoms in the alkyl group.

7. The percutaneous absorption applying sheet according to claim 6, wherein the contents of said organic compound (A), said oxycarboxylic acid and/or dicarboxylic acid (B) and said compound (C) are from 0.1 to 15 parts by weight based on 100 parts by weight of the pressure-sensitive adhesive, respectively.

8. The percutaneous absorption applying sheet according to claim 1, wherein said pressure-sensitive adhesive layer comprises 100 parts by weight of the pressure-sensitive adhesive, 0.1 to 65 parts by weight of the acid salt of morphine and 0.1 to 45 parts by weight of the percutaneous absorption enhancer.

9. The percutaneous absorption applying sheet according to claim 1, wherein said pressure-sensitive adhesive is a (meth)acrylic pressure-sensitive adhesive.

10. The percutaneous absorption applying sheet according to claim 9, wherein said pressure-sensitive adhesive comprises a copolymer of alkyl (meth)acrylate and N-vinyl-2-pyrrolidone as monomer units.

11. The percutaneous absorption applying sheet according to claim 10, wherein said pressure-sensitive adhesive comprises a copolymer composed of 65 to 99 parts by weight of 2-ethylhexyl acrylate, 1 to 35 parts by weight of N-vinyl-2-pyrrolidone and 0 to 0.5 part by weight of a polyfunctional monomer.

12. The percutaneous absorption applying sheet according to claim 9, wherein said pressure-sensitive adhesive comprises a plurality of alkyl (meth)acrylates as monomer units.

13. The percutaneous absorption applying sheet according to claim 12, wherein said pressure-sensitive adhesive comprises a copolymer composed of 100 parts by weight of 2-ethylhexyl methacrylate, 5 to 30 parts by weight of 2-ethylhexyl acrylate, 5 to 30 parts by weight of dodecyl methacrylate and 0 to 0.5 part by weight of a polyfunctional monomer.

14. The percutaneous absorption applying sheet according to claim 9, wherein said pressure-sensitive adhesive incorporates a tackifier.

15. The percutaneous absorption applying sheet according to claim 14, wherein said tackifier is a material selected from the group consisting of rosin resin, terpene resin, cumarone-indene resin, petroleum resin and terpene-phenol resin or a mixture thereof.

16. The percutaneous absorption applying sheet according to claim 15, wherein said tackifier is glycerin ester of hydrogenated rosin.

17. The percutaneous absorption applying sheet according to any one of claims 1 to 16, wherein said acid salt of morphine is morphine hydrochloride or morphine sulfate.

18. The percutaneous absorption applying sheet according to claim 1, wherein said log P value of percutaneous absorption enhancer is from -0.3 to 1.8.

19. The percutaneous absorption applying sheet according to claim 2, wherein said aliphatic alcohol having 4 to 6 carbon atoms is a compound selected from the group consisting of butyl alcohol, isobutyl alcohol, t-butyl alcohol, pentyl alcohol, isopentyl alcohol and hexyl alcohol or a mixture thereof.

20. The percutaneous absorption applying sheet according to claim 2, wherein said aliphatic monocarboxylic acid having 2 to 5 carbon atoms is a compound selected from the group consisting of acetic acid, propionic acid, butyric acid and valeric acid or a mixture thereof.

21. The percutaneous absorption applying sheet according to any one of claims 4 to 7, wherein said oxycarboxylic acid is a compound selected from the group consisting of lactic acid, glyceric acid, tartaric acid and citric acid or a mixture thereof.

22. The percutaneous absorption applying sheet according to any one of claims 4 to 7, wherein said dicarboxylic acid is a compound selected from the group consisting of oxalic acid, malonic acid, succinic acid, glutaric acid, adipic acid, pimelic acid, suberic acid, fumaric acid, maleic acid, phthalic acid, isophthalic acid, terephthalic acid and malic acid or a mixture thereof.

23. The percutaneous absorption applying sheet according to claim 6 or 7, wherein said aliphatic acid amide is a compound selected from the group consisting of capric acid di(mono)ethanol amide, lauric acid di(mono)ethanol amide and palmitic acid di(mono)ethanol amide or a mixture thereof.

24. The percutaneous absorption applying sheet according to claim 6 or 7, wherein said acyl sarcosine is a compound selected from the group consisting of N-lauroyl sarcosine, coconut fatty acid sarcosine, N-stearoyl sarcosine, N-oleoyl sarcosine and N-palmitoyl sarcosine or a mixture thereof.

25. The percutaneous absorption applying sheet according to claim 6 or 7, wherein said alkyl hydroxybenzoate is a compound selected from the group consisting of methyl p-(o- or m-)hydroxybenzoate, ethyl p-(o- or m-)-hydroxybenzoate, propyl p-(o- or m-)hydroxybenzoate, isopropyl p-(o- or m-)hydroxybenzoate and butyl p-(o- or m-)hydroxybenzoate or a mixture thereof.

26. The percutaneous absorption applying sheet according to any one of claims 1 to 25, which is packaged in a packaging container made of a sheet or a film having an oxygen permeability of not more than 100 cm³/m² · atm · 24 hrs (25°C) and said sheet or film being selected from the group consisting of an aluminum sheet, an aluminium sheet of which surface is covered with polyethylene or polyethylene terephthalate, a polyvinylidene chloride film and a polyvinylidene chloride-polyethylene laminated film.
